# EUROPEAN PATENT APPLICATION

(11) **EP 2 338 875 A1**
(43) Date of publication of application: **29.06.2011**
(21) Application number: 09179805.8
(22) Date of filing: 18.12.2009
(51) Int. Cl.: C07C 227/16, C07C 229/36

(54) **Process for the preparation of thyroid hormones and derivatives thereof**

(71) Applicant: Bracco Imaging S.p.A, 20134 Milano (IT)
(72) Inventor: Viscardi, Carlo Felice, 1-10010, Ivrea (IT); Cappelletti, Enrico, 1-10010, Ivrea (IT); Gazzetto, Sonia, 1-10010, Ivrea (IT); Galimberti, Laura, 1-10010, Ivrea (IT); Sini, Loredana, 1-10010, Ivrea (IT)
(74) Representative: Macchetta, Francesco

(57) **Abstract**

The present invention generally refers to a process for the preparation of L-thyroxine derivatives. More in particular, the present invention relates to a iodination reaction of an aromatic derivative with an appropriate iodinating agent, so to afford the related iodinated compound as disodium salt, which may represent a useful intermediate for the synthesis of the L-thyroxine mono-sodium salt, and the free form thereof.

## Description

The present invention relates in general to a process for the preparation of thyroid hormones and derivatives thereof. More in particular, it relates to a iodination reaction of an aromatic substrate with an appropriate iodinating agent, so to afford the related iodinated compound, which may represent a useful intermediate for the synthesis of hormonal derivatives.

### Background

Hormonal derivatives are a class of compounds which plays an important biological role in several essential metabolic transformations. Among said class, thyroid hormones, and particularly, iodinated biphenyl compounds such as, for example, thyroxine (or 3,5,3',5'-tetraiodothyronine, often abbreviated as T4), and triiodothyronine (T3), represent important key molecules, being involved in controlling the rate of various metabolic processes in the body. In particular, the mono-sodium salt of both L-thyroxine and L-triiodothyronine are widely employed in the treatment of several pathologies related to the malfunctioning of thyroid.

A variety of processes for the preparation of T4 and T3 have been disclosed in the past, initially by using animal natural sources as starting material (see US 2,889,363), and later on by enzymatic or bio-mimetic synthesis (see US 2,889,364). A further enhancement has been described in WO 96/11904 (Baxter), where an organometallic oxidative coupling is performed in order to obtain the thyroxine hydrochloride derivative, subsequently converted in the corresponding sodium salt, as appropriate.

IT 1302201 (Bracco) disclosed a process for the synthesis of the mono sodium salt of the L-thyroxine with an improved overall yield compared to a similar process known in the art (see e.g. Chalmers et al., J. Chem. Soc. 1949, 3424). According to the prior art teaching, the preparation of the monosodium salt of L-thyroxine occurs, in the most of the cases, by a proper and controlled acidification of the corresponding disodium salt, substantially as per scheme I below:

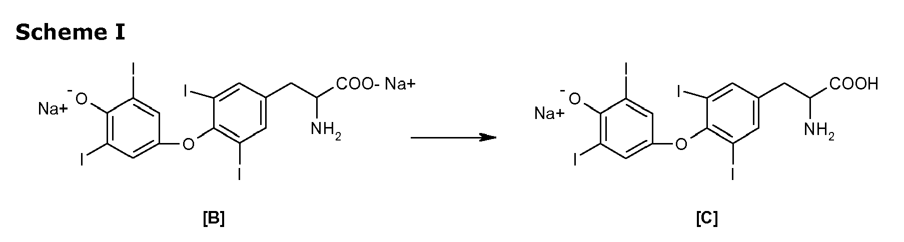

Typically, the reaction may be carried out by reacting the disodium salt with a strong inorganic acid, for instance hydrochloric acid, followed by a proper pH regulation by using an alkaline base, such as Na₂CO₃.

In its turn, the disodium salt formerly indicated as compound **B** may be prepared, for instance, according to IT 1302201 (Bracco), according to the Scheme II below:

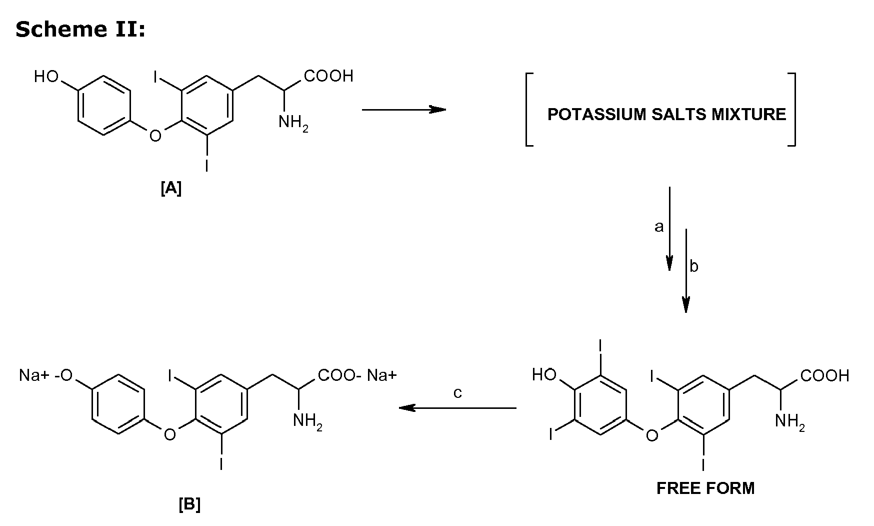

As schematically shown above, compound **A** is reacted with the widely used KI/I₂ system as the iodinate agent of choice (see for instance Taylor et al. The Ohio Journal of Science, Vol. 53, 37-41, 1953), in an aqueous medium and in the presence of a suitable amine.

The so obtained mixture of mono and di-potassium salts (herein general referred as potassium salt mixture) is subjected to a subsequent separation and purification step, including typically:
(a) the addition of an organic acid (e.g. acetic acid) to obtain the corresponding free form;
(b) the separation of the free form (as a precipitate); and
(c) the final addition of an excess of sodium hydroxide to obtain the L-thyroxine disodium salt.

The applicant has now found a new process whereby compound **B** can be directly obtained by a conversion of compound **A,** substantially avoiding the above mentioned supplementary steps.

In particular, we have now found that by using NaI/I₂ in lieu of KI/I₂ as iodinating system, the iodination of compound **A** leads to the tetra iodinated derivative in high yields as mono- and di-sodium salts mixture. Surprisingly, in fact, the obtained sodium salts are substantially insoluble in the reaction medium, and may be easily converted in the desired compound **B** by pH regulation. The thus obtained disodium salt may then be collected as such, or even employed as intermediate as described below.

### Summary of the Invention

The present invention relates in general terms to a new process for the preparation of the sodium salts of thyroidal hormones, and free form thereof, by iodination of a known substrate in the presence of NaI and I₂.

According to a first aspect, the present invention relies on a process for the preparation of the compound of formula **B,** 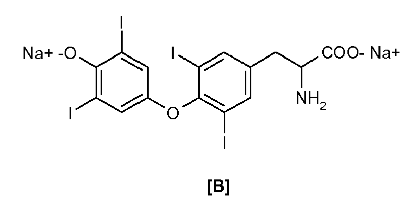 which comprises reacting compound of formula **A** 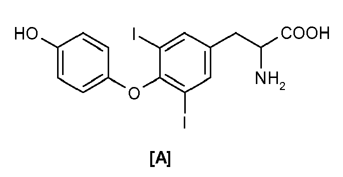 with a iodinating agent comprising NaI and I₂, in the presence of an aliphatic amine.

The thus obtained compound **B** may be collected and stored according to methodologies known ion the prior art.

Furthermore, the L-thyroxine disodium salt thus obtained, may be readily used in the preparation of the corresponding mono-sodium salt **C,** accordingly for instance to the prior art teachings (see IT 1302201).

According to another aspect of the invention, the present process allows for the preparation of compound **C** starting from **B** by means of a reliable basification/acidification step as extensively reported in the experimental part herein below. By that, the L-thyroxine monosodium salt may be obtained with higher overall yields, respect to the prior art.

Remarkably, as compound **B** is an optical active one, the process of the present invention allow for its preparation by starting from the corresponding optical active isomer, by fully retaining the configuration at the stereocentre during the course of the reaction. It will be clear to the skilled in the art that any optical isomers of compound B (i.e. the (S) and the (R) form, as well as any racemic mixture thereof) are to be intended as included in the scope of the present invention.

### Detailed description of the Invention

The iodinating agent of the present invention may be prepared by solving NaI and a proper amount of I₂ in distilled water. Such an aqueous solution (hereinafter referred to as the "NaI/I₂ system"), may be advantageously prepared either at the moment of use (i.e. just before the iodination reaction), or beforehand (*e.g.* from few to several hours before) and kept at room temperature before its use. According to an embodiment of the invention, the molar ratio between NaI and I₂ in the iodinating agent is from 2 to 3, preferably from 2.40 to 2.70.

Practically, compound **A** is suspended in water in the presence of a proper organic amine, and the NaI/I₂ system is subsequently added thereto over about 3h obtaining the product precipitation. Suitable amines may be, for instance, aliphatic (C₁-C₄) amines, in particular linear mono (C₁-C₄) amines including, *inter alia* methylamine, ethylamine, propylamine and mixtures thereof, being ethylamine more preferred. The addition is generally carried out at room temperature, which is of about 20° C. Once the iodination is completed, the reaction mixture is further elaborated and the pH is adjusted to a basic value, preferably around 8-12, by addition of a base. Among the bases which can be used in this respect, an aqueous solution of the commonly inorganic bases such as alkaline metal hydroxides, carbonates, and the like may be conveniently employed, whereas aqueous NaOH is preferred, and aqueous Na₂CO₃ is even more preferred.

The so obtained solid is re-dissolved in a mixture of ethanol, water and NaOH, so that compound **B** will represent the main reaction mixture component. In particular, the disodium salt may hence be collected, e.g. by filtration, in high yields and with a high degree of purity and eventually stored, for instance, under moisture safe conditions.

Alternatively, and as formerly mentioned, compound **B** may be treated as precursor of either the L-thyroxine mono-sodium salt (compound **C),** or L-thyroxine free form (compound **D),** 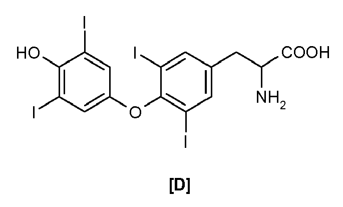 by a proper pH regulation and subsequent precipitation of the product of choice.

In this direction, compound **C,** may be obtained from **B,** for instance, according to the method disclosed in IT1302201 (Bracco), which basically consisted in adjusting the pH to a value of about 10, by addition of Na₂CO₃, to an acidic solution of **B** (*i.e.* by increasing the pH), at high temperature.

Remarkably, when compound **B** is obtained according to the present invention, the final compound **C** is obtained in higher overall yields, compared to those described in the prior art.

Furthermore, we have now found that compound **C** may be obtained by precipitation in even higher yields by performing the pH regulation step by adding an acid, such as on organic weak acid, to an alkaline aqueous solution of **B** (*i.e.* by decreasing the pH), at high temperature.

Therefore, it is a further aspect of the present invention a process for the preparation of compound **C** comprising the addition at high temperature of a suitable acid to an alkaline aqueous solution of compound **B,** the latter preferably obtained as formerly reported, so to adjust the pH to a value of about 10. The pH may be monitored by means of a pH electrode equipment or by any other conventional method.

With the term "alkaline aqueous solution of compound **B"** we mean a solution containing such compound in an alkaline base aqueous solution such as, e.g. aqueous Na₂CO₃ or NaOH.

In this respect, an alkaline aqueous solution of **B** is heated at a selected temperature, and the acid of choice is added dropwise. A representative example of a proper acid may be any suitable inorganic or organic acid, preferably a weak organic acid such as, for example acetic acid. Particularly, the acid is added at a temperature of the solution comprised between about 70° C and 95° C, preferably between about 75° C and 85° C and even more preferably, between about 82° C and 85° C.

After cooling to room temperature (i.e. 20 °C, or even below), over a frame of time of about 3h, the precipitated L-thyroxine mono-sodium salt may be collected, for instance, by filtration, in a proper solid form, in very high yields.

The filter cake may be optionally washed with water, or eventually with aqueous ethanol, to facilitate the recovering of the solid.

It is worth noting that the water content of the alkaline solution throughout the precipitation process may be chosen for example from the minimum content required for the solubilisation of the reagents, to even higher amounts, without substantial variations in the overall yield, as herein reported in the Table 1. In particular, such a water content may be suitably selected from about 11 to about 25 w/w, (whereas w/w means amount of total water with respect to the amount of compound **B)** whereas a water content that lies between 11 and 15w/w is preferred.

Therefore, according to a preferred embodiment of the invention, compound C may be obtained by adjusting the pH to a value of about 10 by addition of acetic acid to an alkaline solution of compound **B,** at a temperature of 82-85°C and with a water content of the reaction mixture between 11 to 15 w/w.

As previously mentioned, the present process may be employed also in the preparation of the L-thyroxine free form (compound **D),** by adjusting the pH of an aqueous solution of compound **B** (or even a solution of compound **C)** to values lower than 8, preferably lower than 6.

Example of suitable pH regulating agent known for this purpose may be, among others, any proper acid, e.g., hydrochloric acid or acetic acid, being the latter preferred.

The starting material of the present process is known in the art, and may be prepared in accordance with conventional methods. Likewise, any additional reactants or solvents are known and commonly adopted in organic synthesis.

From all the above, it can be concluded that the process of the invention enables for the preparation of L-thyroxine di-sodium salt (compound **B)** by reacting compound **A** with an iodinating agent comprising NaI and I₂, advantageously without isolating any intermediate. The method described in the present invention provides, advantageously, an increasing in the yields and a reduction in the reaction times when compared to a similar method which contemplates the metal alkaline equivalent KI/I₂ system as iodinating agent.

As extensively reported, the compound **B** thus obtained may be efficiently employed in the synthesis of the L-thyroxine mono-sodium salt, according to the prior art teaching or, alternatively and more advantageously, according to the formerly reported procedure.

Furthermore, the present process enables for the preparation of the L-thyroxine free form in a very convenient and reliable procedure.

It will be apparent to the skilled person that certain changes and modifications may be practiced within the scope of the appended claims and that the present embodiments are to be considered as illustrative and not restrictive.

Accordingly, the following examples are herein intended to better illustrate the process of the present invention, without posing any limitation to it.

### Experimental section

### Example 1: preparation of L-thyroxine disodium salt (compound B) by reaction of compound A with the NaI/I₂ system.

Compound A (1.0 kg) and NaI (approx. 0.3 kg) were suspended in water (approx. 8.5 kg), under nitrogen atmosphere, and 70% aq monoethylamine (approx. 5.6 kg) was added in about 1 h keeping the temperature below 22°C, obtaining a solution. A solution of I₂ (approx. 1.1 kg) and NaI (approx. 1.3 kg) in water (approx. 5.0 kg) was added over about 3 h maintaining the temperature below 22°C (product precipitates). The suspension was stirred for 1 h and then a solution of Na₂SO₃ (approx. 0.1 kg) and Na₂CO₃ (approx. 0.5 kg) in water (approx. 2.8 kg) was added. After stirring for 15 min, the mixture was heated to 50-65°C, obtaining a solution, and water/monoethylamine were distilled under reduced pressure. The mixture was cooled to about 20°C and acetic acid (approx. 0.3 kg) was added until pH 10. The suspension was then cooled to 10°C, filtered and the solid washed with water (approx. 4.8 kg).

Wet solid was suspended in water (approx. 0.8 kg), abs ethanol (approx. 5.3 kg) and 30% NaOH (approx. 0.3 kg). To the mixture, heated to 50-70 °C, more abs ethanol (approx. 3.3 kg) was added. The solution obtained was cooled to 10°C leading to the precipitation of Compound B which was filtered and washed with cold abs ethanol (yield 86%).

### Example 2:

### Preparation of L-thyroxine mono sodium salt (compound C) starting from B as prepared in Example 1

Compound B, obtained according to example 1, was dissolved by adding an aqueous solution of NaOH according to Table 1 at 25-30°C. After addition of Na₂SO₃ (approx. 0.03 kg) and activated charcoal (approx. 3 g), the mixture was stirred for 0.25 h and filtered on Millipore (0.45µm).

The solution was warmed up to about 40-50° C, the ethanol was distilled at reduced pressure, and aqueous Na₂CO₃ (see Table 1 below) was added, to constitute an alkaline solution of compound B.

The alkaline solution was heated to a temperature T1 according to Table 1, and acetic acid was added. The solution was cooled to about 15° C over 3 hours and then gently stirred for about 0.5 h. The solid thus precipitated was filtered off, and the filter cake was washed with water (approx. 3.0 Kg) and EtOH aq.

Wet sodium levothyroxine was dried at approximately 35°C under vacuum, giving the desired dried powder.

**Table 1: operative conditions (example2).**

| **Entry** | **H₂O** **(w/w) ^{a}** | **NaOH** **(eq. mol.)^{b}** | **Na₂CO₃** **(eq. mol.)^{b}** | **CH₃COOH** **(eq. mol.)^{b}** | **T1** **(°C)** | **Yield ^{c}** **(%)** |
|---|---|---|---|---|---|---|
| **1.** | 11 | 0,50 | 7,43 | 2,42 | 80 | 88 |
| **2.** | 11 | 0,42 | 6,32 | 2,06 | 82-85 | 83 |
| **3.** | 13 | 0,50 | 7,43 | 2,90 | 80 | 90 |
| **4.** | 13 | 0,49 | 7,43 | 2.42 | 82-85 | 88 |
| **5.** | 13,5 | 0,44 | 6,69 | 2,18 | 82-85 | 86 |
| **6.** | 15 | 0,50 | 7,43 | 2,42 | 70-75 | 84 |
| **7.** | 15 | 0,49 | 7,43 | 2,42 | 75-80 | 82 |
| **8.** | 15 | 0,49 | 7,43 | 2,42 | 82-85 | 89 |
| **9.** | 15 | 0,50 | 7,43 | 2,42 | 85-90 | 81 |
| **10.** | 15 | 0,49 | 7,43 | 2,42 | 90 | 83 |
| **11.** | 15 | 0,50 | 7,43 | 2,42 | 90-95 | 85 |
| **12.** | 16,5 | 0,54 | 8,18 | 2,66 | 82-85 | 83 |
| **13.** | 17,5 | 0,49 | 7,43 | 2,42 | 85 | 84 |
| **14.** | 17,5 | 0,50 | 7,43 | 2,42 | 90 | 86 |
| **15.** | 19 | 0,49 | 14,87 | 3,35 | 90-95 | 85 |
| **16.** | 20 | 0,49 | 7,43 | 2,42 | 70-75 | 76 |
| **17.** | 20 | 0,50 | 7,43 | 2,42 | 75-80 | 81 |
| **18.** | 20 | 0,49 | 7,43 | 2,42 | 82-85 | 84 |
| **19.** | 20 | 0,50 | 7,43 | 2,42 | 85-90 | 83 |
| **20.** | 20 | 0,50 | 7,43 | 2,42 | 90 | 89 |
| **21.** | 25 | 0,49 | 7,43 | 2,42 | 82-85 | 79 |
| | | | | | | |
| a) amount of total water / amount of compound B b) mol of reactant / mol of compound B c) referred to Sodium Levothyroxine pentahydrate | | | | | | |

## Claims

1. A process for the preparation of a thyroid hormone derivative, which comprises reacting a compound of formula **A,** with a iodinating agent comprising NaI and I₂, in the presence of an aliphatic amine, to obtain a compound of formula **B.**

2. A process according to claim 1 wherein the aliphatic amine is ethylamine.

3. A process according to claims 1-2, further comprises adding an organic or inorganic acid to an alkaline aqueous solution of **B,** to obtain compound **C.**

4. A process according to claim 3 wherein the acid is acetic acid.

5. A process according to claim 3-4 wherein the acid is added to the alkaline aqueous solution of **B** at a temperature between 70 and 95°C.

6. A process according to claim 5 wherein the acid is added to the alkaline aqueous solution of **B** at a temperature between 82°C and 85°C.

7. A process according to claim 3-6 wherein the water content of the alkaline aqueous solution of **B** is selected from 11 w/w to 25 w/w.

8. A process according to claim 7 wherein the water content of the alkaline aqueous solution of **B** is selected from 11 w/w to 15 w/w.

9. A process according to claim 3-8 wherein the acid is added to the alkaline aqueous solution of **B** at a temperature between 75°C and 85°C, and said solution has a content of water comprised from 11 to 15 w/w.

10. A process according to claim 9 wherein the acid is added to the alkaline aqueous solution of **B** at a temperature between 82°C and 85°C.

11. A process according to claims 1, further comprises adjusting the pH of the solution containing compound **B,** to value lower than 8 to obtain compound **D.**
